Europäisches Patentamt

European Patent Office

Office européen des brevets

(11) Veröffentlichungsnummer: **0 281 778**
**A1**

(12) **EUROPÄISCHE PATENTANMELDUNG**

(21) Anmeldenummer: **88101927.7**

(22) Anmeldetag: **10.02.88**

(51) Int. Cl.⁴: **A61K 9/22**

(30) Priorität: **12.02.87 DE 3704275**
**27.03.87 DE 3710175**

(43) Veröffentlichungstag der Anmeldung:
**14.09.88 Patentblatt 88/37**

(84) Benannte Vertragsstaaten:
**AT BE CH DE ES FR GB GR IT LI LU NL SE**

(71) Anmelder: **HOECHST AKTIENGESELLSCHAFT**
**Postfach 80 03 20**
**D-6230 Frankfurt am Main 80(DE)**

(72) Erfinder: **Deasy, Patrick Bernard, Dr.**
**1 Richview Villas Clonskeagh Road**
**Dublin 14(IE)**

(54) **Mehrteilige implantierbare Arzneizubereitung mit Langzeitwirkung.**

(57) Mehrteilige Implantate, die mindestens zwei wirkstoffhaltige Formlinge enthalten, dadurch gekennzeichnet, daß diese biologisch abbaubare Copolymere von Milchsäure und Glykolsäure mit einem Lactid:Glykolid-Gewichtsverhältnis von 90:10 bis 60:40 enthalten und mindestens zwei Formling-Typen A und B vorliegen, wobei Typ A Copolymere mit einem um 5 bis 15 Gew.-% niedrigeren Gehalt an Lactid als Typ B enthält, setzen den Wirkstoff über einen längeren Zeitraum gleichmäßig oder in zunehmender Abgabemenge ab.

EP 0 281 778 A1

## Mehrteilige implantierbare Arzneizubereitung mit Langzeitwirkung

Gegenstand der Erfindung ist eine mehrteilige implantierbare Arzneizubereitung mit Langzeitwirkung, die biologisch abbaubare wirkstoffhaltige Formlinge und wirkstofffreie Formlinge enthält.

Aus der britischen Patentschrift 1 325 209 sind bereits zur Implantation geeignete pharmazeutische Zubereitungen in Form von Teilchen oder Pellets mit geregelter Wirkstoffabgabe bekannt, bei denen der Wirkstoff in inniger Mischung mit einem festen Polyactid oder einem anderen biologisch abbaubaren Polyester vorliegt. Ferner ist beispielsweise aus EP-A 25698 bekannt, Copolymere von Milchsäure und Glykolsäure zur Herstellung von solchen Zubereitungen zu verwenden. Die Polyester werden langsam abgebaut und geben dabei über einen entsprechend langen Zeitraum den Wirkstoff frei.

Derartige Arzneizubereitungen sind zwar gut verträglich, haben aber den Nachteil, daß sie insbesondere bei länger dauernden Behandlungsperioden keine gleichmäßige und zunehmende Wirkstoff-Freigabe gewährleisten können. Vielmehr wird der in der Matrix dispergierte Wirkstoff infolge der sich durch den allmählichen Abbau des Implantats immer mehr verkleinernden Oberfläche in ständig abnehmenden Mengen freigegeben.

Deshalb ist auch schon versucht worden, durch unterschiedliche Verteilung des Wirkstoffs im Pellet oder durch Beimischung von leicht herauslösbaren Zusatzstoffen eine gleichmäßigere Wirkstoff-Freisetzung zu erreichen. Außerdem wurde versucht, dieses Ziel durch besondere geometrische Gestaltungen des Implantats zu erreichen, beispielsweise durch Ausbildung des Implantats als eine dünne Folie oder als Hohlfaser. Dabei hat sich aber gezeigt, daß biologisch abbaubare aliphatische Polyester auch nach Zusatz von Weichmachern noch so brüchig sind, daß dünne Folien und Hohlfasern zur Herstellung von Implantaten, die mittels einer grobkanüligen Injektionsapparates unter der Haut deponiert werden sollen, ungeeignet sind. Solche Ausformungen von Implantaten erfordern den Einsatz von biologisch nicht abbaubaren Elastomeren wie Siliconkautschuk, wenn sie nicht schon während der Implantation oder nach Ablage unter der Haut alsbald zerbrechen sollen.

Gegenstand der Erfindung ist ein mehrteiliges Implantat, das mindestens zwei wirkstoffhaltige Formlinge enthält, dadurch gekennzeichnet, daß diese biologisch abbaubare Copolymere von Milchsäure und Glykolsäure mit einem Lactid: Glykolid-Gewichtsverhältnis von 90:10 bis 60:40 enthalten und mindestens zwei Formling-Typen A und B vorliegen, wobei Typ A Copolymere mit einem um 5 bis 15 Gew.-% niedrigeren Gehalt an Lactid als Typ B enthält.

Lactid und Glykolid sind jeweils die Dimeren von Milch-und Glykolsäure. Es können die reinen Stereoisomeren oder deren Gemische eingesetzt werden.

Durch eine solche Kombination von Formling-Type läßt sich erreichen, daß die Freisetzungsgeschwindigkeit des Wirkstoffes bei gleichmäßiger oder steigender Wirkstoffabgabe über einen längeren Zeitraum (bis zu 12 Monaten) optimal gesteuert werden kann und die Nachteile der bekannten Implantate somit überwunden werden. Das Implantat hat ferner den Vorteil, daß ein sogenannter "burst" Effekt, bei dem zu Beginn eine hohe Menge an Wirkstoff freigesetzt ist, minimiert wird.

Die Herstellung von Copolymeren aus Milchsäure und Glykolsäure ist aus EP-A-26599 und D.L.Wise et al. in Drug Carriers in Biology and Medicine, Part 3, S. 237-270 (1979), Ed. Gregoriadif Academic Press, bekannt.

Das Implantat kann bis zu 20 wirkstoffhaltige Formlinge, insbesondere 5 bis 15 Formlinge enthalten, insbesondere eine ungerade Anzahl von Formlingen, die in einer kettenförmigen oder sandwich-artigen Anordnung kombiniert sind. Vorzugsweise sind 1 bis 7 Formlinge des Typs B und 2 Formlinge des Typs A enthalten, wobei ein Formling A jeweils an den beiden Kettenenden orientiert ist.

Die für das erfindungsgemäße Implantat benötigten Formlinge haben im allgemeinen zylindrische Form mit einem Durchmesser von 2 bis 6 mm, vorzugsweise von 3 bis 4 mm und einer Dicke (= Höhe) von 1 bis 6 mm, vorzugsweise 2 bis 4 mm. Die Gesamtlänge des Implantats kann vorzugsweise zwischen 1 und 4 cm variieren. Die Formlinge werden vorzugsweise durch Zusammenpressen eines Gemisches, das den Wirkstoff, das biologisch abbaubare Copolymer und andere geeignete Zusatzstoffe wie Gleitmittel enthält, zwischen den flachen Stempeln einer Tablettenpresse hergestellt.

Die Wirkstofffreigabe aus den Formlingen des Implantates läßt sich durch verschiedene Parameter erheblich beeinflussen. Die Erhöhung des Molekulargewichts des Polyesters verzögert seinen Abbau und die Freisetzung des Wirkstoffes. Innerhalb der Polymilchsäure-und Polyglykolsäurepolymeren und -copolymeren nimmt bei gleichem Molekulargewicht die Abbaugeschwindigkeit von der Poly-L-Milchsäure über die Poly-DL-Milchsäure, die Polymilchglykolsäure bis zur Polyglykolsäure zu. Eine Erhöhung der Wirkstoffmenge in dem Formling erhöht die Freisetzungsrate ebenso wie ein Zusatz von Weichmachern oder von leicht herauslösbaren Zusatzstoffen. Ebenso kann durch Erhöhung der Zahl der Formlinge oder Vergrößerung ihrer Oberfläche die Wirkstoffreigabe beschleunigt werden. Dagegen wirken sich eine Erhöhung des Pressdruckes oder eine Behandlung der Presslinge durch Anwendung erhöhter Temperaturen hemmend auf die Freisetzung des Wirkstoffes aus.

Das mittlere Molekulargewicht ( $\overline{M}$n) der zu verwendenden Copolymere kann zwischen 10 000 und 30 000, die Polydispersität zwischen 1,5 und 2,5 variieren. Die Formlinge A und B können vor der Applikation untereinander verklebt werden; hierfür eignen sich als Klebemittel solche, die bioabbaubar sind, wie Cyanoacrylate, z.B. Methyl, Ethyl-oder Butylcyanoacrylat.

Durch eine sorgfältige Abstimmung der Zusammensetzung der wirkstoffhaltigen Formlinge läßt sich das Wirkstoffabgabe-Verhalten der Implantate steuern, so daß je nach Wirkstoffart die Freisetzungsdauer variiert werden kann.

In weiterer Ausgestaltung der Erfindung können die erfindungsgemäßen Implantate wirkstofffreie Formlinge vom Typ C enthalten, die zwischen die oben beschriebenen wirkstoffhaltigen Formlinge eingefügt werden können. Diese Formlinge können dazu dienen, das Abgabeverhalten des Implantats über einen längeren Zeitraum zu verbessern. Die Formlinge vom Typ C sind zweckmäßigerweise aus denselben Copolymeren, wie sie für die wirkstoffhaltigen Formlinge A und B beschrieben sind, aufgebaut.

Die Copolymere der Formlinge C können 50 bis 70 Gew.-% Lactid und 50 bis 30 Gew.-% Glykolid enthalten.

Der Wirkstoffgehalt in den Formlingen A oder B variiert zwischen 20 und 80 Gew.-% in Bezug auf das Gewicht der Formlinge, wobei der Wirkstoffgehalt in den Formlingen A vorzugsweise 5 bis 15 Gew.-% niedriger ist als in den Formlingen B.

Die Formlinge A, B oder C, insbesondere die Formlinge C können inerte Füllmaterialien enthalten, die die Kompression der Formlinge bei deren Herstellung erleichtern. Solche Materialien sind beispielsweise $MgCO_3$, MgO, $CaCO_3$, $CaHPO_4$. Der Gewichtsanteil dieser Materialien in dem Formling C kann zwischen 30 und 50 Gew.-% betragen.

Die erfindungsgemäßen Implantate können vor allem in der Veterinärmedizin verwendet werden, eignen sich aber auch für den Einsatz in der Humanmedizin wenn es erforderlich ist, über einen längeren Zeitraum eine gleichmäßige oder zunehmende Arzneimittelkonzentration im Organismus zu gewährleisten. Vor allem bei hormonalen Störungen, zur Krebsbehandlung, zur Behandlung von Infektionen, Kreislaufstörungen, psychischen Defekten, zur Geburtenkontrolle bieten sich derartige Implantate mit Langzeitwirkung an.

In der Veterinärmedizin können solche Implantate eingesetzt werden zur Behandlung von Mangelzuständen (Vitamin-, Spurenelementmangel), chronischen Infektionen (Langzeitapplikation von Antiinfektiva), Ekto-und Endoparasitosen und Unterfunktion oder Fehlregulationen endokriner Organe (hormonelle Substitutionen) ebenso wie zur gleichmäßigen Freisetzung von Substanzen oder natürlichen und synthetischen Hormonen, insbesondere solchen, die das Wachstum beeinflussen.

Natürliche Hormone wie 17 $\beta$-Estradiol und/oder Testosteron oder ihre Ester, aber auch synthetische Hormone wie Trenbolonacetat oder Resorcylsäurelacton (Zeranol) beeinflussen das Wachstum von Kälbern deutlich, wenn sie in Form des erfindungsegemäßen mehrteiligen Implantats verabreicht werden. Da das Implantat den Wirkstoff in Abhängigkeit von den Eigenschaften der hierfür ausgewählten Formlinge über einen Zeitraum von 3 bis 12 Monaten abgeben kann, ist im Gegensatz zu bekannten Implantaten nur eine einzige Implantation für jedes Tier erforderlich.

Folgende Wirkstoffe können bevorzugt eingesetzt werden:

Steroide oder andere Stoffe mit anaboler Wirkung, wie Trenbolon, Zeranol, Estradiol-17$\beta$, Testosteron, Progesteron oder Kombinationen hiervon, Peptidhormone oder Substanzen, die Peptidhormone freisetzen, wie Somatotropin, das Somatotropin-Freisetzungshormon oder das Gonadotropin-Freisetzungshormon.

Die fertigen Implantate werden mit Hilfe eines handelsüblichen Implatationsgeräte direkt unter die Haut deponiert.

Nachfolgende Beispiele dienen zur Herstellung der Erfindung.

Beispiele

**a) Herstellung der Copolymere aus Milchsäure und Glykolsäure**

Die käuflichen Dimeren Lactid und Glykolid von Milchsäure bzw. Glykolsäure wurden dreimal aus Essigester umkristallisiert und anschließend bei 60°C 24 Stunden getrocknet. Für das 80/20 Copolymere wurden 4 g des L(-)Lactids und 1 g Glykolid in eine vorgetrocknete Polymerisationsröhre (Quickfit MF 24-3) zusammen mit 0,03 Gew.-% Zinn-Octanoat und 0,01 Gew.-% Laurylalkohol, beide in Hexan gelöst, gegeben. Für das 50/50 Copolymere wurden jeweils 2,5 g der Dimeren verwendet.

3

Das Reaktionsgefäß wurde in einem Silikonölbad für 4,5 Stunden bei 200°C unter vermindertem Druck erhitzt, wobei mittels eines Magnetrührers gerührt wurde.

Danach wurde das Reaktionsgemisch abgekühlt; das erhaltene Copolymere wurde bei Raumtemperatur in einem Exsikkator aufbewahrt.

Das Copolymer (80 Gew.-% Lactid, 20 % Glykolid) hatte folgende Eigenschaften: Molekulargewicht $\bar{M}$n: 22470, Polydispersität: 2,26. Auf dieselbe Weise wurden die Copolymeren mit anderem Lactid/Glycolid-Gehalt hergestellt. Das Copolymer mit 70 Gew.-% Lactid/30 Gew.-% Glykolid hatte ein Molekulargewicht $\bar{M}$n: 16900 und eine Polydispersität: 1,76.

## b) Herstellung der Formlinge

Für die wirkstoffhaltigen Formlinge wurden 180 mg 17 β-Estradiol mit 120 mg 80/20 Copolymeren aus Lactid/Glykolid vermischt und in Aceton gelöst. Dann wurde das Lösungsmittel im Vakuum abdestilliert. Es wurden Tabletten mittels einer Tablettenpresse aus dem erhaltenen Material hergestellt. Analog erfolgte die Herstellung der wirkstofffreien Formlinge.

## c) Biologische Beispiele

### Implantat I

Das Implantat bestand aus 5 wirkstoffhaltigen Formlingen in Form von zylindrischen Tabletten: 3 Tabletten wiesen einen höheren Wirkstoffgehalt auf (Typ B) und besaßen einen Durchmesser von 4 mm und eine Dicke von 2 mm. Die 2 anderen Tabletten mit gleicher Dimension wiesen einen geringeren Lactid-Anteil auf (Typ A) und wurde mit den Tabletten von Typ B in einer kettenförmigen Anordnung kombiniert, in der Reihenfolge: ABBBA.

Die Tabletten B wiesen einen Copolymeren-Anteil von 40 Gew.-% auf bei einem Lactid/Glykolid-Verhältnis von 80/20 und einem Wirkstoffgehalt an 17β-Estradiol von 60 Gew.-%. Die Tabletten A bestanden zu 50 Gew.-% aus Copolymeren mit einem Lactid/Glykolid-Verhältnis von 70/30 und zu 50 Gew.-% aus 17 β-Estradiol.

### Implantat II

Das Implantat bestand aus 9 Formlingen in Form von zylindrischen Tabletten. Zusätzlich zum Implantat I enthielt Implantat II 4 wirkstofffreie Formlinge (Typ C) von gleicher Dimension wie die Tabletten A oder B. Die Tabletten C wurden zwischen die wirkstoffhaltigen Tabletten A und B in folgender Reihenfolge eingefügt: A-C-B-C-B-C-B-C-A.

Die Tabletten C bestanden zu 60 % aus Copolymeren mit einem Lactid/Glykolid-Verhältnis 70/30 und zu 40 Gew.-% aus Magnesiumcarbonat.

### In Vivo-Versuch

Die Abgaberate von 17β-Estradiol aus den oben beschriebenen Implantaten I und II wurde durch Bestimmung des Blutplasmaspiegels vor, während und nach der Implantation an kastrierten männlichen Rindern bestimmt. Außerdem wurde der Effekt der Gewichtszunahme durch Bestimmung des Körpergewichts der Tiere vor, während und nach der Implantation untersucht.

### Material und Methoden

13 Ochsen wurden in 3 Gruppen eingeteilt: Zwei Gruppen von je 5 Tieren (Gruppe I und II) und eine Gruppe mit 3 Tieren (Gruppe III).

Gruppe I (Durchschnittsgewicht 145,2 kg) erhielt Implantat I
Gruppe II (Durchschnittsgewicht 144,8 kg) erhielt Implantat II

Gruppe III (Durchschnittsgewicht 141,3 kg) erhielt kein Implantat = Kontrolle

Die Implantation wurde mit einem handelsüblichen Applikator durchgeführt. Das Implantat wurde in der Dorsalseite der Ohrmuschel der Tiere eingesetzt.

42 Tage nach der Implantation wurden die Implantate von zwei Tieren aus der Gruppe I und II entfernt und der verbleibende Estradiol-Gehalt des Implantats durch HPLC-Analyse bestimmt. 84 Tage nach der Implantation wurden die Implantate der verbleibenden 3 Tiere jeder Gruppe I und II entfernt und analysiert.

Zu bestimmen Zeitpunkten vor, während und nach der Implantation wurden von den getesteten Tieren Blutproben entnommen, um den Plasmaspiegel von Estradiol festzustellen. Der Estradiol-Gehalt wurde radioimmunologisch bestimmt.

Außerdem wurden die Versuchstiere im Abstand von 2 Wochen nach der Implantation gewogen, um die Gewichtszunahme zu ermitteln.

Jedem Tier wurde pro Fütterung während des Versuchs eine bestimmte Futtermenge zugemessen und die Aufnahme kontrolliert.

**Ergebnisse**

Die Estradiol-Abgabemenge für das Implantat I und II nach 42 und 84 Tagen ist in Tabelle 1 angegeben. Der Plasmaspiegel von Estradiol ist in Tabelle 2 aufgeführt. Aus Tabelle 3 ist die Gewichtszunahme der Tiere ersichtlich.

Aus den Ergebnissen der Tabellen 1 bis 3 läßt sich eindeutig ersehen, daß die Implantate I und II eine Abgaberate von Estradiol über einen Zeitraum von mindestens 12 Wochen gewährleisten, wobei die Gewichtszunahme höher ist als bei der Kontrolle.

**Tabelle 1**

Freisetzung von Estradiol-17β(E-17β)

| Implantat | E-17β-Gehalt (mg) pro Implantat vor der Implantation | E-17β-Gehalt nach | | Freisetzung von E-17β mg/Tag über | |
|---|---|---|---|---|---|
| | | 42 Tagen | 84 Tagen | 42 Tage | 84 Tage |
| Gruppe I | 78,80/77,06 | 72,0/64,0 | | 0,16/0,31 | |
| | 76,29/76,63/ 75,55 | | 41,4/35,6/ 45,6 | | 0,42/0,49/ 0,39 |
| Gruppe II | 77,94/79,09 | 68,5/54,0 | | 0,22/0,60 | |
| | 79,23/79,47/ 77,96 | | 45,1/49,9 48,3 | | 0,41/0,35/ 0,35 |

0 281 778

**Tabelle 2**

Estradiol-Plasmaspiegel

| | Tage | Grupppe I (pg/ml) | Gruppe II (pg/ml) | Gruppe III (pg/ml) |
|---|---|---|---|---|
| | | (n = 5) | (n = 5) | (n = 3) |
| | -5 | --- | 11,7 | 15,8 |
| | -4 | 14,9 | 16,5 | 21,0 |
| Implantat-Applikation | 0 | 21,0 | 31,0 | 32,3 |
| | 1 | 92,6 | 83,5 | 16,0 |
| | 2 | 36,0 | 42,6 | 20,7 |
| | 7 | 69,5 | 50,8 | - 20,1 |
| | 14 | 36,6 | 62,6 | 21,1 |
| | 21 | 44,8 | 35,0 | 13,4 |
| | 29 | 21,5 | 25,0 | 23,0 |
| | 35 | 22,5 | 12,9 | 10,4 |
| | 42 | 19,5 | 30,9 | 23,0 |
| | | (n = 3) | (n = 3) | |
| | 49 | 25,4 | 32,2 | 22,3 |
| | 56 | 24,0 | 21,0 | --- |
| | 63 | 38,0 | 58,3 | 27,7 |
| | 70 | 53,7 | 46,0 | 18,3 |
| | 77 | 68,7 | 58,3 | 25,3 |
| Implantat-Entfernung | 84 | 160,0 | 125,0 | 26,3 |
| | 85 | 180,0 | 130,0 | --- |
| | 86 | 110,0 | 93,3 | --- |
| | 90 | 69,0 | 30,0 | 31,3 |

Die Werte beziehen sich auf pg/ml Plasma und entsprechen Durchschnittswerten ($\bar{X}$)

n = Zahl der Tiere

## Tabelle 3

### Gewichtsentwicklung (kg) nach der Implantation

| Gruppe | 0.-2. | 0.-4. | 0.-6. | 0.-8. | 0.-10. | 0.-12. Woche |
|--------|-------|-------|-------|-------|--------|--------------|
| I | 21,4 | 44,2 | 62,3 | 80,0 | 99,3 | 113,3 |
| II | 21,4 | 45,0 | 57,6 | 69,9 | 85,6 | 105,6 |
| III | 18,3 | 35,0 | 43,3 | 59,0 | 70,3 | 81,3 |
| (Kontrolle) | | | | | | |

**Ansprüche**

1. Mehrteiliges Implantat mit Langzeitwirkung, das mindestens zwei wirkstoffhaltige Formlinge enthält, dadurch gekennzeichnet, daß diese biologisch abbaubare Copolymere von Milchsäure und Glykolsäure mit einem Lactid:Glykolid-Gewichtsverhältnis von 90:10 bis 60:40 enthalten und mindestens zwei Formling-Typen A und B vorliegen, wobei Typ A Copolymere mit einem um 5 bis 15 Gew.-% niedrigeren Gehalt an Lactid als Typ B enthält.

2. Implantat nach Anspruch 1, dadurch gekennzeichnet, daß es bis zu 20 Formlinge enthält.

3. Implantat nach Anspruch 1 oder 2, dadurch gekennzeichnet, daß es eine ungerade Zahl von Formlingen enthält.

4. Implantat nach Ansprüchen 1 bis 3, dadurch gekennzeichnet, daß die Formlinge kettenförmig angeordnet sind.

5. Implantat nach Anspruch 4, dadurch gekennzeichnet, daß ein Formling vom Typ A an beiden Kettenenden orientiert ist.

6. Implantat nach Ansprüchen 1 bis 5, dadurch gekennzeichnet, daß es zusätzlich wirkstofffreie Formlinge, die Copolymere aus Milchsäure und Glykolsäure gemäß Anspruch 1 enthalten, aufweist.

7. Implantat nach Anspruch 6, dadurch gekennzeichnet, daß die Formlinge in alternierender Reihenfolge angeordnet sind.

8. Implantat nach Ansprüchen 1 bis 7, dadurch gekennzeichnet, daß der Wirkstoffgehalt in den Formlingen A und B zwischen 20 und 80 Gew.-% variiert.

9. Implantat nach Ansprüchen 1 bis 8, dadurch gekennzeichnet, daß der Wirkstoffgehalt in Formling A 5 bis 15 Gew.-% niedriger ist als in Formling B.

10. Implantat nach Ansprüchen 1 bis 9, dadurch gekennzeichnet, daß es einen Wirkstoff für human-oder veterinärmedizinische Zwecke enthält.

11. Implantat nach Ansprüchen 1 bis 10, dadurch gekennzeichnet, daß es als Wirkstoff ein natürliches oder synthetisches Hormon für Tiere enthält.

Patentansprüche für die folgenden Vertragsstaaten: ES, GR.

1. Mehrteiliges Implantat mit Langzeitwirkung, das mindestens zwei wirkstoffhaltige Formlinge enthält, dadurch gekennzeichnet, daß diese biologisch abbaubare Copolymere von Milchsäure und Glykolsäure mit einem Lactid:Glykolid-Gewichtsverhältnis von 90:10 bis 60:40 enthalten und mindestens zwei Formling-Typen A und B vorliegen, wobei Typ A Copolymere mit einem um 5 bis 15 Gew.-% niedrigeren Gehalt an Lactid als Typ B enthält.

2. Implantat nach Anspruch 1, dadurch gekennzeichnet, daß es bis zu 20 Formlinge enthält.

3. Implantat nach Anspruch 1 oder 2, dadurch gekennzeichnet, daß es eine ungerade Zahl von Formlingen enthält.

4. Implantat nach Ansprüchen 1 bis 3, dadurch gekennzeichnet, daß die Formlinge kettenförmig angeordnet sind.

5. Implantat nach Anspruch 4, dadurch gekennzeichnet, daß ein Formling vom Typ A an beiden Kettenenden orientiert ist.

6. Implantat nach Ansprüchen 1 bis 5, dadurch gekennzeichnet, daß es zusätzlich wirkstofffreie Formlinge, die Copolymeer aus Milchsäure und Glykolsäure gemäß Anspruch 1 enthalten, aufweist.

7. Implantat nach Anspruch 6, dadurch gekennzeichnet, daß die Formlinge in alternierender Reihenfolge angeordnet sind.

8. Implantat nach Ansprüchen 1 bis 7, dadurch gekennzeichnet, daß der Wirkstoffgehalt in den Formlingen A und B zwischen 20 und 80 Gew.-% variiert.

9. Implantat nach Ansprüchen 1 bis 8, dadurch gekennzeichnet, daß der Wirkstoffgehalt in Formling A 5 bis 15 Gew.-% niedriger ist als in Formling B.

10. Verfahren zur Herstellung eines nach mindestens einem der Ansprüche 1 bis 9 definierten Implantats, dadurch gekennzeichnet, daß man mindestens zwei Typen A und B von wirkstoffhaltigen Formlingen kombiniert, die biologisch abbaubare Copolymere von Milchsäure und Glykolsäure mir einem Lactid: Glykolid-Gewichtsverhältnis von 90:10 bis 60:40 enthalten und wobei Typ A Copolymere mit einem um 5 bis 15% niedrigeren Gehalt an Lactid als Typ B enthält.

11. Verfahren zur Verwendung des nach mindestens einem der Ansprüche 1 bis 9 definierten Implantats, dadurch gekennzeichnet, daß es zur gleichmäßigen Freisetzung von Wirkstoffen im tierischen oder menschlichen Organismus eingesetzt wird.

Europäisches
Patentamt

**EUROPÄISCHER RECHERCHENBERICHT**

Nummer der Anmeldung

EP 88 10 1927

## EINSCHLÄGIGE DOKUMENTE

| Kategorie | Kennzeichnung des Dokuments mit Angabe, soweit erforderlich, der maßgeblichen Teile | Betrifft Anspruch | KLASSIFIKATION DER ANMELDUNG (Int. Cl.4) |
|---|---|---|---|
| X,Y | EP-A-0 058 481 (IMPERIAL CHEMICAL INDUSTRIES) * Seite 6, Zeilen 1-24; Seite 10, Zeile 16 - Seite 11, Zeile 20; Seite 15, Zeile 10 - Seite 16, Zeile 7 * --- | 1-11 | A 61 K 9/22 |
| D,Y | EP-A-0 025 698 (ELI LILLY) * Seite 3, Zeile 1 - Seite 5, Zeile 14; Ansprüche * --- | 1-11 | |
| Y | WO-A-8 203 174 (MERCK) * Ansprüche * --- | 1-11 | |
| Y | EP-A-0 171 907 (WAKO) * Ansprüche * ----- | 1-11 | |
| | | | **RECHERCHIERTE SACHGEBIETE (Int. Cl.4)** A 61 K |

Der vorliegende Recherchenbericht wurde für alle Patentansprüche erstellt

| Recherchenort | Abschlußdatum der Recherche | Prüfer |
|---|---|---|
| DEN HAAG | 08-06-1988 | GOETZ G. |